# EUROPEAN PATENT SPECIFICATION

(11) **EP 3 490 632 B1**
(45) Date of publication and mention of the grant of the patent: **09.10.2024**
(21) Application number: 17837450.0
(22) Date of filing: 31.07.2017
(51) Int. Cl.: A61M 1/14, A61H 9/00, A61M 16/16, A61H 33/14, A61M 37/00, G01N 33/50

(54) **SYSTEM FOR HYPOBARIC OXYGENATION WITH MEMBRANE OXYGENATOR**
SYSTEM ZUR HYPOBARISCHEN OXYGENIERUNG MIT MEMBRANOXYGENATOR
SYSTÈME D'OXYGÉNATION HYPOBARIQUE AVEC OXYGÉNATEUR À MEMBRANE

(30) Priority: 01.08.2016 US 201662369278 P
(43) Date of publication of application: 05.06.2019
(60) Divisional application: 24197330.4
(73) Proprietor: Gipson, Keith, Wallingford, Connecticut 06492 (US)
(72) Inventor: Gipson, Keith, Wallingford, Connecticut 06492 (US)
(74) Representative: Petraz, Gilberto Luigi
(86) International application number: PCT/US2017/044572
(87) International publication number: WO 2018/026671

(56) References cited:
- WO-A1-2014/012536
- WO-A1-2015/047927
- WO-A1-2015/047927
- US-A- 5 110 548
- US-A- 5 158 534
- US-A- 5 158 534
- US-A1- 2015 075 526
- US-A1- 2015 202 403

## Description

### CROSS REFERENCE TO RELATED APPLICATION

This application claims priority to U.S. provisional patent application serial number 62/369,278, filed on August 1, 2016.

### FIELD

The disclosure relates to hypobaric oxygenation using membrane oxygenation.

### BACKGROUND

There is a need to replace the function of the heart and lungs by artificial means, such as during heart operations. Also in more chronic disease states, for example during severe pulmonary, cardiac, or renal failure, maintenance of life can be upheld by different artificial means until an organ for transplantation becomes available. In many clinical situations there is a need for an extracorporeal circuit wherein an artificial organ is incorporated.

Gas bubbles are easily formed in blood and are propelled into the circulation of a living being during extracorporeal circulation. Gas bubbles are formed from many sources, including cavitation, temperature gradients, and differences in the amount of gases dissolved between a subject's own and incoming blood, as well as inadvertent physical introduction of gas bubbles into blood by caregivers during surgical manipulation or parenteral administration of fluids. In the case of heart surgery, the extracorporeal circuit contains a gas-exchange device, for example an oxygenator, which is used for oxygenation and removal of carbon dioxide, for example. The close contact between blood and gas in the oxygenator poses significant risks for inadvertent entry of gas bubbles into the circulating blood.

At present, to avoid bubble formation during heart surgery, membrane-type oxygenators are used instead of bubble-oxygenators, high temperature gradients are avoided, and use of suction in the operating field is controlled. Heart-lung machines contain an air bubble sensor that warns the person controlling the heart-lung machine of the appearance of small bubbles, and immediately stops the main pump when larger bubbles appear. Typically, the bubble sensor can discern bubbles with a diameter of approximately 0.3 millimeters (mm) or larger, and stops the main pump when a bubble with a diameter of 3-5 mm is recognized. A system for cardiopulmonary bypass using hypobaric oxygenation is disclosed in document WO-A-2015/047927. This document describes systems for hypobaric oxygenation that employ two flow controllers and one inflow sensor (= pressure sensor), wherein the pressure sensor is placed upstream of both the oxygenatori and the flow controller.

Cardiac surgery is frequently complicated by postoperative neurocognitive deficits that degrade functional capacity and quality of life while increasing healthcare costs. Multifactorial contributors to this significant public health problem likely include gaseous microemboli (GME). The arterial circulation receives thousands of 10-40 micrometer (µm) diameter GME during cardiopulmonary bypass (CPB), despite the use of membrane oxygenation and arterial filtration. Vasooclusive GME cause tissue ischemia and denude endothelium in the brain and other end organs, leading to vascular dilation, increased permeability, activation of platelets and clotting cascades, and recruitment of complement and cellular mediators of inflammation.

Current perfusion practice targets mildly hyperoxic blood gases during CPB, by lowering the partial pressure of oxygen in oxygenator sweep gas by dilution with air, thereby producing the needless side effect of dissolving nitrogen in blood. The blood, thus saturated with dissolved gas, is poorly able to dissolve gases that exist in bubble form as GME.

Nonetheless, there remains a need to prevent or reduce the generation of gas bubbles, e.g., the formation of gas bubbles during heart surgery. In a blood bubble, in the liquid-gas interface, there is an approximately 40-100 Angstroms (Å) (4-10 nanometer) deep layer of lipoproteins that denaturate due to direct contact with the foreign material, e.g., gas. In turn, the Hageman factor is activated, which initiates coagulation and the concomitant adverse consumption of factors promoting coagulation, which are used to prevent bleeding from the surgical wound.

The use of a heart-lung machine for circulatory support in these situations is associated with neurological impairment and other signs of end-organ damage or failure. Several potential mechanisms may be at play, including the delivery of gas emboli from the heart-lung machine into the patient's arterial circulation, which may damage the patient's microvasculature and end-organs. Accordingly, a system for reducing the delivery of gaseous microemboli to patients is needed.

### SUMMARY

Disclosed herein is a system for hypobaric oxygenation using membrane oxygenation according to annexed independent claim 1. Other advantageous features are specified in the annexed dependent claims. The system incorporates a different principle of operation than existing oxygenation systems, which use normobaric (i.e., atmospheric pressure) oxygenation. In an embodiment, the system allows analysis, control, or both, of a flow rate, a fluid composition, a pressure, a temperature, an oxygen fraction, a carbon dioxide fraction, a chemical composition, an anesthetic concentration, an anesthetic partial pressure, or a combination comprising at least one of the foregoing, at any of a number of places in the oxygenation system, including one or more of at the inlet to the oxygenator, at the exit of the oxygenator, at an anesthetic vaporizer, at a sweep gas reservoir inlet or outlet, or a combination comprising at least one of the foregoing. This analysis and control allows for more management of the oxygenation process and improved elimination of gas emboli from blood and other fluids.

### BRIEF DESCRIPTION OF THE FIGURES

The accompanying drawings incorporated in and forming a part of the specification embody several aspects of the present disclosure and, together with the description, serve to explain the principles of this disclosure. In the drawings:
Figure 1 is a schematic representation of an oxygenator that can be used.
Figure 2 is a schematic representation of a hypobaric oxygenation system.
Figure 3 shows an exemplary chamber example for sealing.
Figure 4 shows an exemplary chamber example for sealing.

### DETAILED DESCRIPTION

The following disclosure will detail particular embodiments, which provide methods not falling under the claimed invention and systems for hypobaric oxygenation using a membrane oxygenator An overview of the mechanism and methods used herein is provided. Hypobaric oxygenation aids in reducing the gaseous microemboli (GME) in a fluid system.

As used herein, "fluid" and "blood" are used interchangeably, except as otherwise noted. It is to be understood that the systems and methods can be used for blood, or other fluids, such as a blood substitute, artificial blood, a blood product, such as plasma, platelets, albumin, plasma concentrates, or crystalloids such as saline, lactated ringer's solution, normosol, plasmalyte, hetastarch, or other fluid. It is to be understood that the systems and methods can be used for fluids that are gases, such as oxygen, air, carbon dioxide, or other gases. Gases are included in the definition of "fluid", unless otherwise specified. A fluid can be a liquid that contains dissolved gas.

The fluid source can be from a subject, such as an animal, for example a human, or other mammal. The fluid source can be a fluid reservoir, or from a source outside a subject. The subject can be a patient undergoing treatment or evaluation.

The amount and flow characteristics of the fluid coming from the fluid source into the oxygenator and out of the oxygenator, such as pressure, flow velocity, or flow volume, into the system can be controlled by a flow controller, such as a needle valve with or without electronic control, a mass flow controller, a solenoid flow controller, pump, clamp, partial occlusion clamp, or other flow controller or flow restrictor. A pump may be a centrifugal pump or a roller occlusion pump, as known in the art. A flow controller can be controlled independently, or can be controlled by a controller configured to control other aspects of the system, as described further herein. In an embodiment, at least one of the inflow flow controller, oxygenator inflow flow controller, and the optional outflow flow controller is a mass flow controller configured to receive a signal from the controller.

In an embodiment, the inflow flow controller and the oxygenator inflow flow controller are regulated to maintain a constant sweep gas supply to the oxygenator and to allow the vaporizer between them to function at nearly atmospheric pressure. In an embodiment, an anesthetic vaporizer is used in the systems. An anesthetic vaporizer is not always present, for example, in ECMO circuits.

In order for the system of flow controllers to maintain a constant sweep gas supply at a near-atmospheric pressure for operation of the vaporizer, it may be desirable or necessary to employ a sweep gas reservoir between the inflow flow controller and the oxygenator inflow flow controller. A sweep gas reservoir is an optional component. The reservoir may be fully elastic, fully rigid, or partially rigid and partially elastic. The reservoir can be capable of containing oxygen, nitrogen, carbon dioxide, and anesthetic vapor in isolation from the surrounding room air. The reservoir can be of any size necessary to allow the proper functioning of the sweep gas handling system, as its purpose is to cushion temporary discrepancies between the flows through the inflow flow controller and the oxygenator inflow flow controller. If used, the sweep gas reservoir or the connections between the inflow flow controller and the oxygenator inflow flow controller can contain a pressure meter, a negative pressure alarm, a positive pressure relief valve, and a negative pressure relief valve. Since the anesthetic output of the vaporizer is dependent on its pressure of operation, close control of the pressure in this part of the system is warranted. In an embodiment, the negative pressure alarm and negative pressure relief valve are triggered at or near -50 mmHg relative to ambient pressure. In an embodiment, the negative pressure alarm is a whistle or other suitable electronic or mechanical device attached to the negative pressure relief valve. In an embodiment, the negative pressure relief valve is attached to a supplemental oxygen source to avoid unwanted entrainment of room air. In an embodiment, the positive pressure relief valve opens at or near +50 mmHg relative to ambient pressure.

A sensor is used to measure one or more characteristics of the fluid in the system. A sensor can be used in any desirable or useful location in the system to determine a flow rate, a fluid composition, a pressure, a temperature, hematocrit, a chemical composition, an anesthetic concentration, an anesthetic partial pressure, the concentration of anticoagulants or other medication, concentration of gases, such as oxygen, nitrogen, carbon dioxide, air, blood oxygen saturation, or a combination comprising at least one of the foregoing. The system can include one or more sensors configured to measure one or more characteristics. These sensors can be any of a number of common sensors, known to one of ordinary skill in the art.

In embodiments, a sensor is introduced at a location after the fluid source, and a sensor is introduced at a location before the fluid passes into the oxygenator, and an optional sensor is introduced at a location after the fluid passes from the oxygenator. The same sensor can be used, as known in the art, where the fluid path can include separately the inflow and the outflow, or a portion therein. A filter can be used in the system at any appropriate location to remove any desired substance, such as bubbles, particulates, particular gases such as carbon dioxide, or any other desired substance, as known to one of ordinary skill in the art. A filter can be made from any suitable material, such as polyester. A carbon dioxide absorbent could be any suitable substance such as sodalime, baralyme, or amosorb, and may be used as a part of a circle or recirculation system of sweep gas.

The oxygenator can be any of a number of devices, such as a membrane oxygenator, a diffusion membrane oxygenator, or a hollow fiber microporous membrane oxygenator. In an embodiment, the oxygenator is a microporous membrane oxygenator with a sealed housing. The connections of the fluids to and from the oxygenator, to and from the sweep gas source, to and from the venous reservoir, and to and from the subject, and other connections are those known by one of ordinary skill in the art without undue experimentation. The operation of the oxygenator is known by one of ordinary skill in the art without undue experimentation.

In an embodiment, the oxygenator is configured to have a subatmospheric pressure. Commercially available oxygenators are air-tight between the sweep gas inlet and the upstream end of the sweep gas fiber bundle, herein called the sweep gas inlet manifold, and application of subatmospheric pressures here is achievable without modification. From the downstream end of the sweep gas fiber bundle to the sweep gas outlet, herein called the sweep gas outlet manifold, commercially available oxygenators typically have one or more vent holes for pressure relief. The occlusion of these vent holes is essential to the successful application of subatmospheric pressure to the oxygenator fiber bundle. The occlusion of the vent holes is difficult to achieve in many oxygenator models. Any suitable method may be used to provide a sealed housing for the oxygenator. In an embodiment, a commercial membrane oxygenator is used, where some of the existing ports are closed, or sealed, for example, with putty, wax, or stoppers. In an embodiment, a complex array of vents is occluded by a specially fitted belt of medical grade material, such as silicone, rubber, or plastic that does not interfere with attachments of the oxygenator with other devices. In an embodiment, the housing of the oxygenator is sealed using an externally applied chamber resembling a boot or box of medical grade material, such as silicone, plastic, polycarbonate, polymer, glass, metal, or rubber. In an embodiment, the chamber allows air-tight entry and exit ports for necessary attachments, including one or more of blood entry and exit tubes, sweep gas entry and exit tubes, water heat exchanger entry and exit tubes, physical attachment to the blood reservoir, heart-lung machine, or IV pole, cardioplegia line, blood sample line, air purge line, temperature sensor cable, capnography sample line, or shunt line. In an embodiment, the chamber encloses the entire oxygenator. In an embodiment, the chamber forms a seal around a relatively smooth portion of the waist or midsection of the oxygenator, or other opportune portion of the oxygenator, and encloses the end of the oxygenator containing the sweep gas outlet manifold and its vent holes. In an embodiment, the chamber allows application of negative pressure to the oxygenator by isolating the vent holes of the oxygenator from the air in the room. In an embodiment, the chamber is fully flexible. In an embodiment, the chamber is fully rigid except as needed to form air-tight seals with necessary components. In an embodiment, the chamber has a combination of rigid and flexible components to facilitate isolation of the vent ports while supporting ease of application and while resisting deformation under vacuum that may threaten some of the oxygenator attachments. In an embodiment, the flexible portions comprise ridges that insert or snap into grooves on the rigid portion to facilitate assembly and resist leaks under vacuum. In an embodiment, the chamber contains one or more positive-pressure relief valves. In an embodiment, the chamber provides a fitting for attaching the vacuum source instead of applying the vacuum source to the sweep gas outlet of the oxygenator. Applying the chamber to some oxygenators may require detachment of the oxygenator from the blood reservoir or other support component of the heart lung machine or equipment. In an embodiment, the chamber includes a fitting to reattach the oxygenator to its support or other component of the heart-lung machine or its equipment. In an embodiment, the fitting involves one or more of a hook, loop, pin, wire, string, chain, elastic band, molded plastic fitting, clamp, clip, adhesive, or a receptacle for a similar fitting on the heart-lung machine or equipment. In an embodiment, the chamber has clear portions to allow viewing of the oxygenator. In an embodiment, the chamber is composed of multiple parts to allow assembly, application, or sealing with the oxygenator and related components. In an embodiment, the chamber employs o-rings, seals, washers, or bushings to facilitate air-tight attachments. In an embodiment, the chamber contains a vacuum gauge. In an embodiment, the chamber has an attachment for an external vacuum gauge. In an embodiment, the oxygenator does not contain micropores.

Once the oxygenator housing is sealed, either directly or by means of a chamber, sidestream analysis of exhaust sweep gases becomes possible. The results of this analysis may be used to control the heart-lung machine by the user or by electronic control in an automated system. In an embodiment, a sidestream port for gas/vapor sampling is fluidly connected to the sweep gas outlet, and can be located on the outlet, on the chamber, on the chamber outlet, or on the vacuum apparatus downstream of the sweep gas outlet, or a combination comprising at least one of the foregoing. In an embodiment, the measurement of oxygen in exhaust sweep gases is used to control the amount of pressure applied to the oxygenator sweep gas compartment or the sweep gas mixture. In an embodiment, the oxygenator uses the sweep gas from one fluid source to oxygenate blood from a different fluid source. In an embodiment, the measurement of carbon dioxide in exhaust sweep gases is used to control the sweep gas flow rate. In an embodiment, the measurement of anesthetic vapor is used to control the anesthetic vaporizer or the amount of anesthetic vapor in the sweep gas. In an embodiment, the system comprises an anesthetic vaporizer. In an embodiment, a measurement of subatmospheric pressure in the oxygenator is used to control the anesthetic vaporizer, the amount of anesthetic vapor in the sweep gas, or the amount of pressure applied to the oxygenator sweep gas compartment, or a combination comprising at least one of the foregoing. In an embodiment, the measured anesthetic vapor is adjusted for the level of subatmospheric pressure in the oxygenator to provide a value of the effective partial pressure of anesthetic vapor, which is in turn used to control the anesthetic vaporizer or the amount of anesthetic vapor in the sweep gas, or the amount of pressure applied to the oxygenator sweep gas compartment, or a combination of at least one of the foregoing. For example, 2% sevoflurane vapor delivered at 0.5 atmospheres absolute pressure is as effective as 1% sevoflurane vapor delivered at atmospheric pressure since the partial pressure of the anesthetic is reduced by the subatmospheric pressure. In an embodiment, the control involves a user reading and reacting to the measured values. In an embodiment, the control involves a computerized control of circuit components and programmable software. In an embodiment, the user may enter target concentrations for concentrations or pressures of oxygen, carbon dioxide, or anesthetic vapor in the software.

It is also possible to control oxygenation parameters using oxygenation values measured in blood or via continuous or intermittent blood gases including measurement of oxygen tension or carbon dioxide tension. In an embodiment, the readout from an oximeter on the arterial or venous return lines from the patient is used to interrupt or adjust the subatmospheric pressure in the fiber bundle in case of oxygen desaturation below a fixed or user-determined level. In an embodiment, the oxygen readout from intermittent or continuous blood gases is used to adjust the subatmospheric pressure in the fiber bundle to achieve oxygenation goals for the patient. In an embodiment, the carbon dioxide readout from intermittent or continuous blood gases is used to adjust the sweep gas flow rate in the fiber bundle to achieve carbon dioxide removal and pH goals for the patient. In an embodiment, the control involves a user reading and reacting to the measured values. In an embodiment, the control involves a computerized control of circuit components and programmable software. In an embodiment, the user may enter target concentrations for concentrations or pressures of oxygen, carbon dioxide, pH, or any other desired parameter in the software.

After the fluid is passed through the oxygenator, the fluid can be stored, or reinfused into a subject or patient, or recirculated through the heart-lung machine, for example.

Parameters of the system can be controlled by a controller, configured to control the inflow flow controller, the oxygenator inflow flow controller, the outflow flow controller, the inflow sensor, the oxygenator inflow sensor, the outflow sensor, or a combination comprising at least one of the foregoing. Each of the parameters of the system can also be controlled independently, such as by use of a manual dial or valve. In an embodiment, the controller comprises a processor and software instructions implemented by the processor. In an embodiment, the controller controls a parameter wirelessly.

In an embodiment, the controller and other electronics are integrated into a device that can be separate from the system. In an embodiment, the controller and other electronics can be integrated into a box or device that can be attached to an I.V. pole, or other location, for example. In an embodiment, the controller can be used remotely, such as at a nurses' station, where a property from a sensor is displayed, and the flow, pressure, or other parameter of fluid into or out of the oxygenator is adjusted in response. In an embodiment, the controller is part of a computer system, where adjustments can be made in a computer program. In an embodiment, the controller is configured to determine the difference between a property measured at the inflow sensor, the oxygenator inflow sensor, or the outflow sensor and is configured to adjust the flow at the inflow flow controller, the oxygenator inflow flow controller, or the outflow flow controller in response to the difference.

In an embodiment, room air, compressed air, oxygen, carbon dioxide, anesthetic, or other gas or vapor is introduced into fluid source, and the concentration of carbon dioxide, or other gas or vapor is measured at the outflow sensor. The amount of carbon dioxide and other gases in the room air is known, and the amount of carbon dioxide and other gases coming from the oxygenator can be calculated, using known relationships, to determine the concentration of gases in the fluid coming from the oxygenator. The fluid source can have a known concentration of gases, or the concentration of gases can be evaluated at the inflow sensor or oxygenator inflow sensor.

In an embodiment, the pressure in the oxygenator is subatmospheric, and can have a pressure of 0.1 to 1 atmospheres absolute. In an embodiment, the system includes a vacuum regulator fluidly connected to the oxygenator, and configured to provide the subatmospheric pressure. In an embodiment, the pressure in the oxygenator is 10 to 100 percent of normal atmospheric pressure. In an embodiment, the concentration of oxygen at the outflow of the oxygenator is 85 to 100%. In an embodiment, the partial pressure of nitrogen at the outlet is zero, near zero, or minimized.

The fluid entering the oxygenator can be filtered, cooled, warmed, fortified with various medications or gases, or otherwise treated. The fluid can be the fluid to be oxygenated, for example, blood, or can be the fluid providing the oxygen, for example sweep gas. In an embodiment, the partial pressure of oxygen at the outflow of the oxygenator is 50 to 800 mmHg. In an embodiment, the partial pressure of oxygen at the outflow of the oxygenator is 100 to 700 mmHg. In an embodiment, the partial pressure of oxygen at the outflow of the oxygenator is 75 to 650 mmHg. In an embodiment, the partial pressure of oxygen at the outflow of the oxygenator is 150 to 250 mmHg. In an embodiment, the partial pressure of carbon dioxide at the outflow of the oxygenator is 0 to 100 mmHg. In an embodiment, the partial pressure of nitrogen at the outlet is zero. In an embodiment, the partial pressure of nitrogen at the outlet is minimized. In an embodiment, the partial pressure of nitrogen at the outlet is a nonzero level up to about 600 mmHg.

The fluid source can be a blender configured to combine one or more fluids. The system can be used for a number of different fluids, such as blood, a blood product, a blood substitute, oxygen, carbon dioxide, anesthetic, air, or a combination comprising at least one of the foregoing. In an embodiment, a blood flows within the system.

In an embodiment, the oxygenator further comprises a condensate exit port or condensation cavity for collection of condensate. In an embodiment, the condensate exit port has a drain hole, or a separate reservoir for water or other condensation. In an embodiment, the condensation cavity is part of the oxygenator housing. In an embodiment, the condensation cavity is near the sweep gas outlet or sweep gas outlet manifold in the oxygenator housing. In an embodiment, the condensation cavity is at a dependent portion (i.e., lower by gravity) of the oxygenator housing. In an embodiment, the condensate exit port occurs at a dependent portion of the condensation cavity. In an embodiment, the condensate exit port is a drain hole with or without an occlusive device such a stopper that can be manually opened and closed by an operator. In an embodiment, the condensate exit port has a luer-lock connection for attachment of a syringe or tube. In an embodiment, the condensate exit port includes a 3-way stopcock to allow the user to drain the condensation cavity or sweep gas outlet manifold or oxygenator housing. In an embodiment, the sweep gas outlet is placed in a dependent portion of the oxygenator housing to allow escape of condensate into a condensation cavity or into the vacuum tubing or apparatus. In an embodiment, the condensation cavity contains a pierceable diaphragm to allow condensate to be removed using a syringe and needle. In an embodiment, the pierceable diaphragm is made of rubber, silicone, plastic, polymer, or other suitable substance.

In an embodiment, the oxygenator further comprises a positive pressure relief valve, which is fluidly connected to an outlet of the oxygenator. A positive pressure relief valve allows for relief of pressure in the system above a set limit, especially in the case of vacuum failure, for example. In an embodiment, a positive pressure relief valve is a boot flapper valve. In an embodiment, the positive pressure relief valve is a small orifice in the oxygenator connected fluidly to the sweep gas outlet that permits venting of positive pressure at near-atmospheric pressures and usual sweep gas flow rates while not inhibiting the generation of negative pressure from the vacuum source which is capable of higher flow rates. This orifice may also be connected fluidly to the sweep gas outlet as part of a device external to the oxygenator. This orifice may also exist in a number, shape, or location to minimize risk of its intentional or accidental occlusion.

A one-way, check, or pressure relief valve may employ a bird-beak, clapper, or ball-valve design or any other suitable design that allows pressure relief at 0-10 liters per minute (L/min) flow rates at pressures of 0-15 millimeters of mercury (mmHg), for example, above ambient (atmospheric) pressure.

A one-way valve may be incorporated into the oxygenator housing. There may be more than one such valve to prevent accidental or intentional occlusion or failure.

Also provided is a method not falling under the claimed invention of providing anesthesia, the method comprising: providing a subatmospheric pressure in a membrane oxygenator system; introducing an anesthesia-containing fluid to the oxygenator via a pressure regulator and a flow restrictor; and controlling a partial pressure of the anesthesia between 0 and 2-4 minimum alveolar concentration (MAC) of the vapor-based anesthetic to provide anesthesia. In an embodiment, sevoflurane is used as the anesthesia. In an embodiment, the method is used to control the partial pressure of sevoflurane between 0 and 60 mmHg. In an embodiment, isoflurane is used as the anesthesia. In an embodiment, the method is used to control the partial pressure of isoflurane between 0 and 40 mmHg. In an embodiment, desflurane is used as the anesthesia. In an embodiment, the method is used to control the partial pressure of desflurane between 0 and 100 mmHg. In an embodiment, halothane is used as the anesthesia. In an embodiment, the method is used to control the partial pressure of halothane between 0 and 30 mmHg.

In an embodiment of the method not falling under the claimed invention, the membrane oxygenator system comprises a fluid source; an inflow flow controller fluidly connected to the fluid source, wherein the inflow flow controller is configured to control the flow rate of the fluid from the fluid source; an inflow sensor configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the fluid source; an oxygenator fluidly connected to the fluid source, wherein the oxygenator is configured to have a subatmospheric pressure, and wherein the oxygenator is configured to oxygenate blood and other fluids from a separate fluid source; an optional sweep gas reservoir fluidly connected to the fluid source and the oxygenator; an oxygenator inflow sensor fluidly connected to the oxygenator, the oxygenator inflow sensor configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the sweep gas reservoir; an oxygenator inflow flow controller fluidly connected to the sweep gas reservoir or an anesthetic vaporizer or a fluid source, wherein the oxygenator inflow flow controller is configured to control the flow rate of fluid from the sweep gas reservoir or the anesthetic vaporizer or the fluid source; an optional outflow sensor fluidly connected to the oxygenator, the outflow sensor configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the oxygenated fluid; an optional outflow flow controller fluidly connected to the oxygenator, wherein the outflow flow controller is configured to control the flow rate of fluid from the oxygenator; a controller configured to control the inflow flow controller, the oxygenator inflow flow controller, or the outflow flow controller, or a combination comprising at least one of the foregoing, in response to a measurement from the inflow sensor, the oxygenator inflow sensor, the outflow sensor, or a combination comprising at least one of the foregoing.

The system and method not falling under the claimed invention can be used with any cardiopulmonary bypass (CPB) system or circuit, including the system described in WO2015/047927, for example. In an embodiment, the system is used in an oxygenation/ventilation system, for example, where oxygen is added to blood, and carbon dioxide is removed. One such oxygenation/ventilation system is an extracorporeal membrane oxygenation (ECMO) system.

Reference is now made to the drawings, wherein like reference numerals are used to refer to like elements throughout the disclosure.

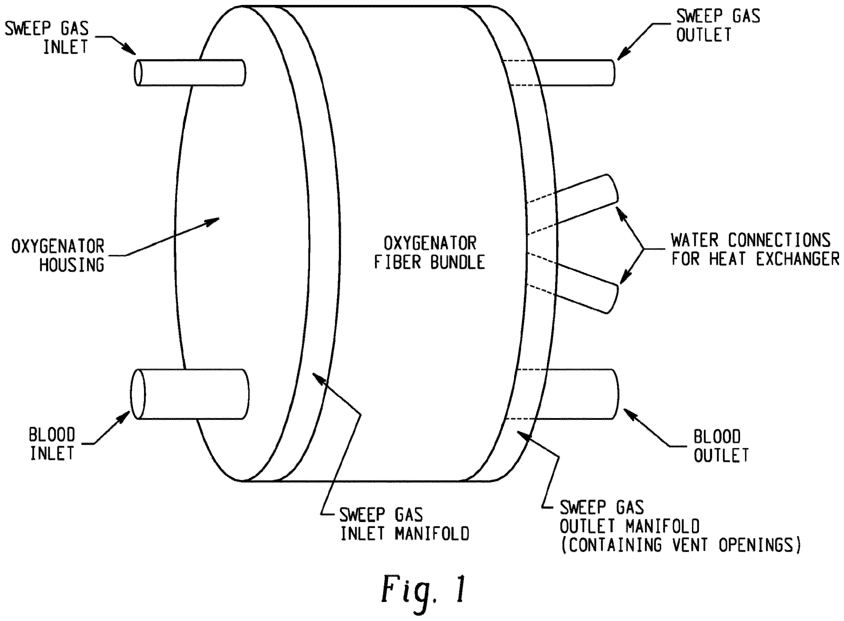

Figure 1 illustrates an exemplary oxygenator that can be used. In an embodiment, one or more ports of the oxygenator can be sealed, plugged, or covered with a material as described further herein, such as a boot, to allow a subatmospheric pressure to be developed.

In Figure 1 illustrates an exemplary oxygenator that can be used. In Figure 1, the oxygenator is shown with a housing, a blood inlet and blood outlet, a sweep gas inlet and outlet, and a sweep gas inlet manifold and sweep gas outlet manifold (containing vent openings). The oxygenator is shown having a fiber bundle. Water connectors for heat exchange are also shown. The oxygenator can use any suitable membrane, such as polypropylene, or other material, as known to one of ordinary skill in the art.

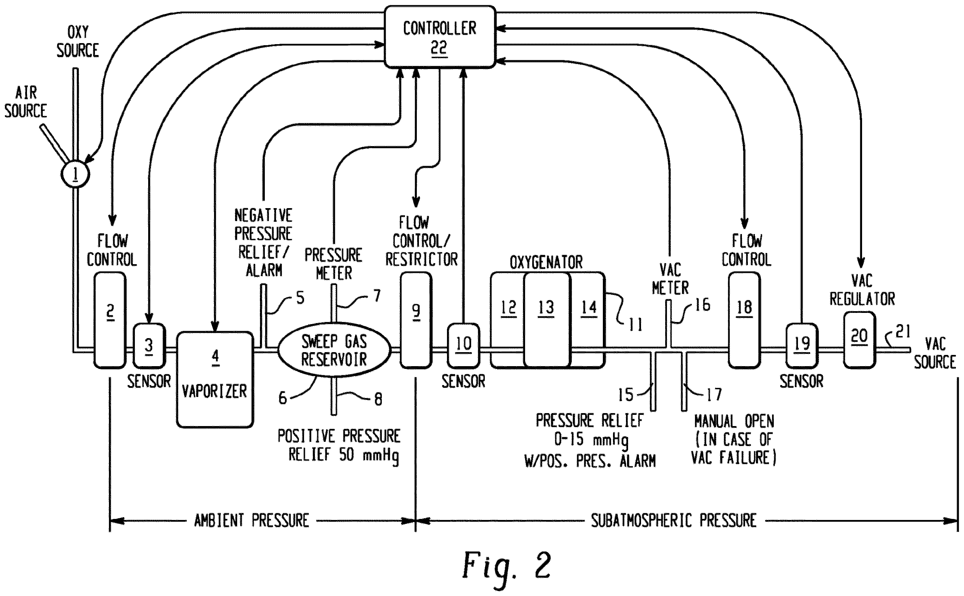

Figure 2 shows a schematic of an exemplary embodiment of an oxygenation system using membrane oxygenation. Fluid (which can be gas, liquid, dissolved gas, or a combination comprising at least one of the foregoing) from fluid source 1 passes into optional inflow flow controller 2 which controls the flow rate, amount, and other characteristics of fluid from fluid source 1 into inflow sensor 3. Fluid from inflow sensor 3 passes into optional vaporizer 4, where it receives anesthetic vapor and increases in volume, then into optional sweep gas reservoir 6. This portion of the circuit contains a negative pressure relief valve 5 with or without alarm, pressure meter 7, and positive pressure relief valve 8. Oxygenator inflow flow controller 9 contains a flow restrictor to allow a pressure drop as seep gas flows past sensor 10 and into oxygenator 11, which includes sweep gas inlet manifold 12, membrane fiber bundle 13, and sweep gas outlet manifold 14. Oxygenator 11 can be any of a number of apparatus or systems useful for adding oxygen to a fluid and removing carbon dioxide. The volume, flow rate, or other parameters of fluid from oxygenator 11 can be sensed by outflow sensor 19. The flow of fluid from oxygenator 11 can be controlled by outflow flow controller 18. The portion of the circuit between membrane fiber bundle 13 and outflow flow controller 18 contains one or more positive pressure relief valves 15 with or without alarm, a vacuum meter 16, and a manual or automatic opening valve 17 in case of vacuum failure. Vacuum regulator 20 attaches a vacuum source 21 to provide subatmospheric pressure to the oxygenator. Aspects of the system, including inflow flow controller 2, inflow sensor 3, oxygenator inflow flow controller 9, oxygenator inflow sensor 10, outflow sensor 19, outflow flow controller 18, vaporizer 4, vacuum regulator 20, and aspects of oxygenator 11 can be controlled by controller 22. Although the inflow sensor is shown in line after the inflow flow controller, and the oxygenator inflow sensor is shown in line after the oxygentor inflow flow controller, and the outflow sensor is shown in line after the outflow flow controller in Figure 2, it is understood that these components can be reversed, i.e., the inflow sensor can be in line before the inflow flow controller, and the oxygenator inflow sensor can be in line before the oxygenator inflow flow controller, and the outflow sensor can be in line before the outflow flow controller, or any combination thereof. Although controller 22 is shown as physically connected to various aspects of the system in Figure 2, it is understood that controller 22 can be wirelessly connected to one or more components of the system.

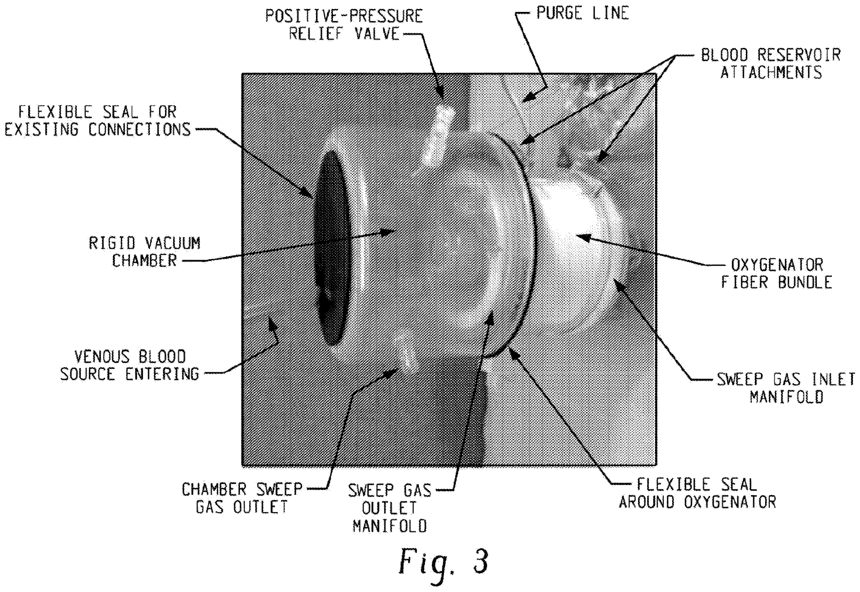

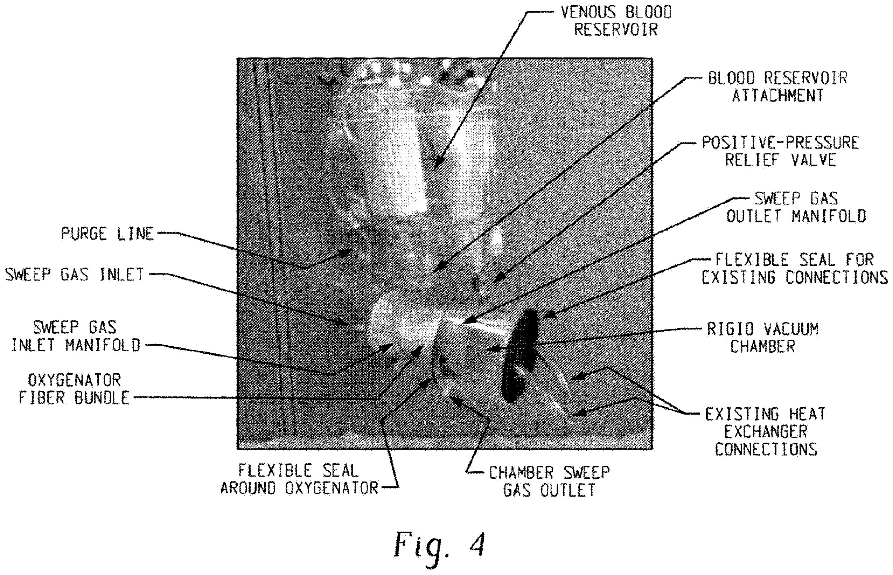

Figures 3 and 4 show exemplary oxygenator chambers. Figure 3 shows a venous blood source entering a rigid vacuum chamber. A flexible seal for the exiting connections is shown. The chamber sweep gas outlet, positive-pressure relief valve, and sweep gas outlet manifold are shown. A flexible seal around the oxygenator is shown. A purge line and blood reservoir attachments are shown. The oxygenator fiber bundle is seen, with the sweep gas inlet manifold.

Figure 4 shows a different oxygenator. A venous blood reservoir is shown attached via a blood reservoir attachment to the oxygenator. A purge line is shown. A sweep gas inlet, sweep gas inlet manifold, and the oxygenator fiber bundle are shown. A flexible seal around the oxygenator is shown. A rigid vacuum chamber is shown attached to the oxygenator, with a chamber sweep gas outlet, sweep gas outlet manifold, and positive-pressure relief valve. A flexible seal for exiting connections, shown as heat exchanger connections is shown.

The system can be provided as a part of an extracorporeal system for oxygenation of blood, where a pump circulates the blood and the oxygenator provides oxygen to the blood and removes carbon dioxide from the blood, and the oxygenated blood can be provided to the subject.

In any of the foregoing embodiments, the oxygenator is a membrane oxygenator, a diffusion membrane oxygenator, or a hollow-fiber microporous membrane oxygenator; and/or the oxygenator is a microporous membrane oxygenator with a sealed housing; and/or the oxygenator comprises one or more openings that may include vent openings from the sweep gas compartment; and/or an opening is closed by a belt, boot or box of medical grade material, a chamber enclosing the oxygenator, or a combination comprising at least one of the foregoing; and/or an opening is occluded by a specially fitted belt of medical grade material, such as silicone, rubber, or plastic that does not interfere with attachments of the oxygenator with other devices; and/or the oxygenator comprises a housing, wherein the housing of the oxygenator is sealed using an externally applied chamber resembling a boot or box of medical grade material, such as silicone, plastic, polycarbonate, polymer, glass, metal, or rubber; and/or the fluid is oxygen, carbon dioxide, anesthetic, air, nitrogen, or a combination comprising at least one of the foregoing; and/or the fluid source is a blender configured to combine one or more fluids; and/or a blood flows within the system; and/or the oxygenator further comprises a condensate exit port or condensation cavity for collection of condensate; and/or the condensate exit port comprises a drain hole, or a separate reservoir; and/or the condensation cavity comprises a pierceable diaphragm; and/or the oxygenator further comprises a positive pressure relief valve, which is fluidly connected to an outlet of the oxygenator; and/or at least one of the inflow flow controller, oxygenator inflow flow controller, and the outflow flow controller is a mass flow controller, solenoid flow controller, needle valve, or other suitable flow controller configured to receive a signal from the controller; and/or the system further comprises a vacuum regulator fluidly connected to the oxygenator, wherein the vacuum regulator is configured to provide the subatmospheric pressure; and/or the controller comprises a processor and software instructions implemented by the processor; and/or the controller is configured to determine the difference between a property measured at two or more of the inflow sensor, the oxygenator inflow sensor, and the outflow sensor and is configured to adjust the flow at one or more of the inflow flow controller, the oxygenator inflow flow controller, or the outflow flow controller in response to the difference; and/or the membrane oxygenator system comprises: a fluid source; an inflow flow controller fluidly connected to the fluid source, wherein the inflow flow controller is configured to control the flow rate of the fluid from the fluid source; an inflow sensor configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the fluid source; an oxygenator fluidly connected to the fluid source, wherein the oxygenator is configured to have a subatmospheric pressure, and wherein the oxygenator is configured to oxygenate blood and other fluids from a separate fluid source; an optional sweep gas reservoir fluidly connected to the fluid source and the oxygenator; an oxygenator inflow sensor fluidly connected to the oxygenator, the oxygenator inflow sensor configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the sweep gas reservoir; an oxygenator inflow flow controller fluidly connected to the sweep gas reservoir or a anesthetic vaporizer or a fluid source, wherein the oxygenator inflow flow controller is configured to control the flow rate of fluid from the sweep gas reservoir or the anesthetic vaporizer or the fluid source; an optional outflow sensor fluidly connected to the oxygenator, the outflow sensor configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the oxygenated fluid; an optional outflow flow controller fluidly connected to the oxygenator, wherein the outflow flow controller is configured to control the flow rate of fluid from the oxygenator; a controller configured to control the inflow flow controller, the oxygenator inflow flow controller, or the outflow flow controller, or a combination comprising at least one of the foregoing, in response to a measurement from the inflow sensor, the oxygenator inflow sensor, the outflow sensor, or a combination comprising at least one of the foregoing.

Also disclosed is a system for hypobaric oxygenation, the system comprising: a fluid source; an inflow flow controller fluidly connected to the fluid source, wherein the inflow flow controller is configured to control the flow rate of the fluid from the fluid source; an inflow sensor configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the fluid source; an oxygenator fluidly connected to the fluid source, wherein the oxygenator is configured to have a subatmospheric pressure, and wherein the oxygenator is configured to oxygenate blood and other fluids from a separate fluid source; an optional sweep gas reservoir fluidly connected to the fluid source and the oxygenator; an oxygenator inflow sensor fluidly connected to the oxygenator, the oxygenator inflow sensor configured to measure one or more of a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the sweep gas reservoir; oxygenator inflow flow controller fluidly connected to the sweep gas reservoir or an anesthetic vaporizer or a fluid source, wherein the oxygenator inflow flow controller is configured to control the flow rate of fluid from the sweep gas reservoir or an anesthetic vaporizer or a fluid source; an optional outflow sensor fluidly connected to the oxygenator, the outflow sensor configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the oxygenated fluid; an optional outflow flow controller fluidly connected to the oxygenator, wherein the outflow flow controller is configured to control the flow rate of fluid from the oxygenator; a controller configured to control the inflow flow controller, the oxygenator inflow flow controller, or the outflow flow controller, or a combination comprising at least one of the foregoing, in response to a measurement from the inflow sensor, the oxygenator inflow sensor, the outflow sensor, or a combination comprising at least one of the foregoing.

Also disclosed is a method not falling under the claimed invention of providing anesthesia, the method comprising: providing a subatmospheric pressure in a membrane oxygenator system; introducing an anesthesia-containing fluid to the oxygenator via a pressure regulator and a flow restrictor; and controlling a partial pressure of the anesthesia between 0 and 2 to 4 minimum alveolar concentration (MAC) of a vapor-based anesthetic to provide anesthesia.

In any of the foregoing embodiments, the oxygenator is a membrane oxygenator, a diffusion membrane oxygenator, or a hollow-fiber microporous membrane oxygenator; and/or the oxygenator is a microporous membrane oxygenator with a sealed housing; and/or the oxygenator comprises one or more openings that may include vent openings from the sweep gas compartment; and/or an opening is closed by a belt, boot or box of medical grade material, a chamber enclosing the oxygenator, or a combination comprising at least one of the foregoing; and/or an opening is occluded by a specially fitted belt of medical grade material, such as silicone, rubber, or plastic that does not interfere with attachments of the oxygenator with other devices; and/or the oxygenator comprises a housing, wherein the housing of the oxygenator is sealed using an externally applied chamber resembling a boot or box of medical grade material, such as silicone, plastic, polycarbonate, polymer, glass, metal, or rubber; and/or the fluid is oxygen, carbon dioxide, anesthetic, air, nitrogen, or a combination comprising at least one of the foregoing; and/or the fluid source is a blender configured to combine one or more fluids; and/or a blood flows within the system; and/or the oxygenator further comprises a condensate exit port or condensation cavity for collection of condensate; and/or the condensate exit port comprises a drain hole, or a separate reservoir; and/or the condensation cavity comprises a pierceable diaphragm; and/or the oxygenator further comprises a positive pressure relief valve, which is fluidly connected to an outlet of the oxygenator; and/or at least one of the inflow flow controller, oxygenator inflow flow controller, and the outflow flow controller is a mass flow controller, solenoid flow controller, needle valve, or other suitable flow controller configured to receive a signal from the controller; and/or the system further comprises a vacuum regulator fluidly connected to the oxygenator, wherein the vacuum regulator is configured to provide the subatmospheric pressure; and/or the controller comprises a processor and software instructions implemented by the processor; and/or the controller is configured to determine the difference between a property measured at two or more of the inflow sensor, the oxygenator inflow sensor, and the outflow sensor and is configured to adjust the flow at one or more of the inflow flow controller, the oxygenator inflow flow controller, or the outflow flow controller in response to the difference; and/or the membrane oxygenator system comprises: a fluid source; an inflow flow controller fluidly connected to the fluid source, wherein the inflow flow controller is configured to control the flow rate of the fluid from the fluid source; an inflow sensor configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the fluid source; an oxygenator fluidly connected to the fluid source, wherein the oxygenator is configured to have a subatmospheric pressure, and wherein the oxygenator is configured to oxygenate blood and other fluids from a separate fluid source; an optional sweep gas reservoir fluidly connected to the fluid source and the oxygenator; an oxygenator inflow sensor fluidly connected to the oxygenator, the oxygenator inflow sensor configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the sweep gas reservoir; an oxygenator inflow flow controller fluidly connected to the sweep gas reservoir or a anesthetic vaporizer or a fluid source, wherein the oxygenator inflow flow controller is configured to control the flow rate of fluid from the sweep gas reservoir or the anesthetic vaporizer or the fluid source; an optional outflow sensor fluidly connected to the oxygenator, the outflow sensor configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the oxygenated fluid; an optional outflow flow controller fluidly connected to the oxygenator, wherein the outflow flow controller is configured to control the flow rate of fluid from the oxygenator; a controller configured to control the inflow flow controller, the oxygenator inflow flow controller, or the outflow flow controller, or a combination comprising at least one of the foregoing, in response to a measurement from the inflow sensor, the oxygenator inflow sensor, the outflow sensor, or a combination comprising at least one of the foregoing.

The use of the terms "a" and "an" and "the" and similar referents in the context of describing the invention (especially in the context of the following claims) is to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Exemplary embodiments of this invention are described herein, including the best mode known to the inventors for carrying out the invention. Variations of those embodiments may become apparent to those of ordinary skill in the art upon reading the foregoing description. The inventors expect skilled artisans to employ such variations as appropriate, and the inventors intend for the invention to be practiced otherwise than as specifically described herein. The invention is defined by the features of the appended claims.

## Claims

1. A system for hypobaric oxygenation, the system comprising:
a fluid source;
an inflow flow controller (2) fluidly connected to the fluid source, wherein the inflow flow controller (2) is configured to control the flow rate of the fluid from the fluid source;
an inflow sensor (3) configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the fluid source;
an oxygenator (11) fluidly connected to the fluid source, wherein the oxygenator (11) is configured to have a subatmospheric pressure, and wherein the oxygenator (11) is configured to oxygenate blood and/or other fluids from a separate fluid source;
an optional sweep gas reservoir (6) fluidly connected to the fluid source and the oxygenator (11);
an oxygenator inflow sensor (10) fluidly connected to the oxygenator (11), the oxygenator inflow sensor (10) configured to measure one or more of a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the sweep gas reservoir when present or from an anesthetic vaporizer or from the fluid source;
an oxygenator inflow flow controller (9) fluidly connected to the sweep gas reservoir (6) when present or to the anesthetic vaporizer or to the fluid source, wherein the oxygenator inflow flow controller 9 is configured to control the flow rate of fluid from the sweep gas reservoir when present or the anesthetic vaporizer or the fluid source, wherein the oxygenator inflow sensor (10) is between the oxygenator (11) and the oxygenator inflow flow controller (9);
an optional outflow sensor (19) fluidly connected to the oxygenator, the outflow sensor (19) configured to measure a flow rate, a fluid composition, a pressure, a temperature, an oxygen fraction, a carbon dioxide fraction, a chemical composition, an anesthetic concentration, an anesthetic partial pressure, or a combination comprising at least one of the foregoing from the oxygenated fluid;
an outflow flow controller (18) fluidly connected to the oxygenator (11), wherein the outflow flow controller is configured to control the flow rate of fluid from the oxygenator (11);
a controller (22) configured to control the inflow flow controller (2,) the oxygenator inflow flow controller (9), or the outflow flow controller (18), or a combination comprising at least one of the foregoing, in response to a measurement from the inflow sensor (3), the oxygenator inflow sensor (10), the outflow sensor (19) when present, or a combination comprising at least one of the foregoing; and
a vacuum source (21) downstream of the oxygenator (11).

2. The system of claim 1, wherein the oxygenator (11) is a membrane oxygenator, a diffusion membrane oxygenator, or a hollow-fiber microporous membrane oxygenator.

3. The system of any of claims 1 to 2, wherein the oxygenator (11) is a microporous membrane oxygenator with a sealed housing.

4. The system of any of claims 1 to 3, wherein the oxygenator (11) comprises a sweep gas outlet manifold that includes vent openings.

5. The system of claim 4, wherein an opening is occluded by a specially fitted belt of medical grade material such as silicone, rubber, or plastic that does not interfere with attachments of the oxygenator with other devices.

6. The system of any of claims 1 to 5, wherein the oxygenator (11) further comprises a housing, wherein the housing of the oxygenator is sealed using an externally applied chamber resembling a boot or box of medical grade material such as silicone, plastic, polycarbonate, polymer, glass, metal, or rubber.

7. The system of any of claims 1 to 6, wherein the fluid is oxygen, carbon dioxide, anesthetic, air, nitrogen, or a combination comprising at least one of the foregoing, and wherein blood flows within the system.

8. The system of any of claims 1 to 7, wherein the fluid source is a blender configured to combine one or more fluids.

9. The system of any of claims 1 to 8, wherein the oxygenator (11) further comprises a condensate exit port or condensation cavity for collection of condensate, wherein the condensate exit port comprises a drain hole, or a separate reservoir, wherein the condensation cavity comprises a pierceable diaphragm.

10. The system of any of claims 1 to 9, wherein the oxygenator (11) further comprises a positive pressure relief valve (15), which is fluidly connected to an outlet of the oxygenator.

11. The system of any of claims 1 to 10, wherein at least one of the inflow flow controller (2), oxygenator inflow flow controller (9), and the outflow flow controller (18) is a mass flow controller, solenoid flow controller, or needle valve, configured to receive a signal from the controller (22).

12. The system of any of claims 1 to 11, further comprising a vacuum regulator fluidly connected to the oxygenator, wherein the vacuum regulator is attached to the vacuum source (21) to provide the subatmospheric pressure.

13. The system of any of claims 1 to 11, wherein the controller (22) is configured to determine the difference between a property measured at two or more of the inflow sensor (3), the oxygenator inflow sensor (10), and the outflow sensor (19), and is configured to adjust the flow at one or more of the inflow flow controller (2), the oxygenator inflow flow controller (9), or the outflow flow controller (18) in response to the difference.

## Patentansprüche

1. System zur hypobaren Oxygenierung, wobei das System umfasst:
eine Fluidquelle;
einen Zustrommengenregler (2), der mit der Fluidquelle in Fluidverbindung steht, wobei der Zustrommengenregler (2) so konfiguriert ist, dass er die Durchflussrate des Fluids von der Fluidquelle regelt;
einen Zustromsensor (3), der so konfiguriert ist, dass er eine Durchflussrate, eine Fluidzusammensetzung, einen Druck, eine Temperatur, einen Sauerstoffanteil, einen Kohlendioxidanteil, eine chemische Zusammensetzung, eine Konzentration eines Anästhetikums, einen Partialdruck eines Anästhetikums oder eine Kombination umfassend mindestens eines der vorgenannten Merkmale von der Fluidquelle misst;
einen Oxygenator (11), der in Fluidverbindung mit der Fluidquelle steht, wobei der Oxygenator (11) so konfiguriert ist, dass er einen Unterdruck aufweist, und wobei der Oxygenator (11) so konfiguriert ist, dass er Blut und/oder andere Fluide von einer separaten Fluidquelle oxygeniert;
ein optionales Spülgasreservoir (6), das in Fluidverbindung mit der Fluidquelle und dem Oxygenator (11) steht;
einen Oxygenator-Zustromsensor (10), der mit dem Oxygenator (11) in Fluidverbindung steht, wobei der Oxygenator-Zustromsensor (10) so konfiguriert ist, dass er eine Durchflussrate, eine Fluidzusammensetzung, einen Druck, eine Temperatur, einen Sauerstoffanteil, einen Kohlendioxidanteil, eine chemische Zusammensetzung, eine Konzentration eines Anästhetikums, einen Partialdruck eines Anästhetikums oder eine Kombination umfassend mindestens eines der vorgenannten Merkmale von dem Spülgasreservoir, wenn dieses vorliegt, oder von einem Anästhesiemittelverdampfer oder von der Fluidquelle misst;
einen Oxygenator-Zustrommengenregler (9), der mit dem Spülgasreservoir (6), wenn dieses vorliegt, oder mit dem Anästhesiemittelverdampfer oder mit der Fluidquelle in Fluidverbindung steht, wobei der Oxygenator-Zustrommengenregler (9) so konfiguriert ist, dass er die Durchflussrate von Fluid von dem Spülgasreservoir, wenn dieses vorliegt, oder dem Anästhesiemittelverdampfer oder der Fluidquelle regelt, wobei sich der Oxygenator-Zustromsensor (10) zwischen dem Oxygenator (11) und dem Oxygenator-Zustrommengenregler (9) befindet;
einen optionalen Abstromsensor (19), der in Fluidverbindung mit dem Oxygenator steht, wobei der Abstromsensor (19) so konfiguriert ist, dass er eine Durchflussrate, eine Fluidzusammensetzung, einen Druck, eine Temperatur, einen Sauerstoffanteil, einen Kohlendioxidanteil, eine chemische Zusammensetzung, eine Konzentration eines Anästhetikums, einen Partialdruck eines Anästhetikums oder eine Kombination umfassend mindestens eines der vorgenannten Merkmale von dem oxygenierten Fluid misst;
einen Abstrommengenregler (18), der mit dem Oxygenator (11) in Fluidverbindung steht, wobei der Abstrommengenregler so konfiguriert ist, dass er die Durchflussrate des Fluids von dem Oxygenator (11) regelt;
eine Steuerung (22), die so konfiguriert ist, dass sie den Zustrommengenregler (2), den Oxygenator-Zustrommengenregler (9) oder den Abstrommengenregler (18) oder eine Kombination umfassend mindestens einen der vorgenannten Regler als Reaktion auf eine Messung von dem Zustromsensor (3), dem Oxygenator-Zustromsensor (10), dem Abstromsensor (19), wenn dieser vorliegt, oder einer Kombination umfassend mindestens einen der vorgenannten Sensoren steuert; und
eine Vakuumquelle (21) stromabwärts des Oxygenators (11).

2. System nach Anspruch 1, wobei der Oxygenator (11) ein Membran-Oxygenator, ein Diffusionsmembran-Oxygenator oder ein mikroporöser Hohlfasermembran-Oxygenator ist.

3. System nach irgendeinem der Ansprüche 1 bis 2, wobei der Oxygenator (11) ein mikroporöser Membran-Oxygenator mit einem abgedichteten Gehäuse ist.

4. System nach irgendeinem der Ansprüche 1 bis 3, wobei der Oxygenator (11) einen Spülgasauslassverteiler umfasst, der Entlüftungsöffnungen enthält.

5. System nach Anspruch 4, wobei eine Öffnung durch einen speziell angepassten Riemen aus medizinischem Material, wie beispielsweise Silikon, Gummi oder Kunststoff, der nicht die Verbindungen des Oxygenators mit anderen Vorrichtungen behindert, verschlossen ist.

6. System nach irgendeinem der Ansprüche 1 bis 5, wobei der Oxygenator (11) ferner ein Gehäuse umfasst, wobei das Gehäuse des Oxygenators mittels einer von außen angebrachten Kammer abgedichtet ist, die einer Manschette oder einem Kasten aus medizinischem Material, wie zum Beispiel Silikon, Kunststoff, Polycarbonat, Polymer, Glas, Metall oder Gummi, ähnelt.

7. System nach irgendeinem der Ansprüche 1 bis 6, wobei das Fluid Sauerstoff, Kohlendioxid, Anästhetikum, Luft, Stickstoff oder eine Kombination umfassend mindestens eines der vorgenannten Fluide ist, und wobei Blut innerhalb des Systems fließt.

8. System nach irgendeinem der Ansprüche 1 bis 7, wobei die Fluidquelle ein Mischer ist, der so konfiguriert ist, dass er ein oder mehrere Fluide vereinigt.

9. System nach irgendeinem der Ansprüche 1 bis 8, wobei der Oxygenator (11) ferner eine Kondensat-Austrittsöffnung oder einen Kondensationshohlraum zum Sammeln von Kondensat umfasst, wobei die Kondensat-Austrittsöffnung eine Abströmöffnung oder ein separates Reservoir umfasst, wobei der Kondensationshohlraum eine durchstechbare Membran umfasst.

10. System nach irgendeinem der Ansprüche 1 bis 9, wobei der Oxygenator (11) ferner ein Überdruckventil (15) umfasst, das in Fluidverbindung mit einem Auslass des Oxygenators steht.

11. System nach irgendeinem der Ansprüche 1 bis 10, wobei mindestens einer von dem Zustrommengenregler (2), dem Oxygenator-Zustrommengenregler (9) und dem Abstrommengenregler (18), ein Massenflussregler, ein Magnetflussregler oder ein Nadelventil ist, die so konfiguriert sind, dass sie ein Signal von der Steuerung (22) empfangen.

12. System nach irgendeinem der Ansprüche 1 bis 11, das ferner einen Vakuumregler umfasst, der mit dem Oxygenator in Fluidverbindung steht, wobei der Vakuumregler mit der Vakuumquelle (21) verbunden ist, um den Unterdruck bereitzustellen.

13. System nach irgendeinem der Ansprüche 1 bis 11, wobei die Steuerung (22) so konfiguriert ist, dass sie die Differenz zwischen einem Merkmal, das an zweien oder mehr von dem Zustromsensor (3), dem Oxygenator-Zustromsensor (10) und dem Abstromsensor (19) gemessen wird, bestimmt und so konfiguriert ist, dass sie den Durchfluss an einem oder mehreren von dem Zustrommengenregler (2), dem Oxygenator-Zustrommengenregler (9) oder dem Abstrommengenregler (18) als Reaktion auf die Differenz einstellt.

## Revendications

1. Un système d'oxygénation hypobare, le système comprenant :
une source de fluide ;
un dispositif (2) de commande de débit d'entrée relié fluidiquement à la source de fluide, le dispositif (2) de commande de débit d'entrée étant configuré pour commander le débit du fluide provenant de la source de fluide ;
un capteur d'entrée (3) configuré pour mesurer un débit, une composition de fluide, une pression, une température, une fraction d'oxygène, une fraction de dioxyde de carbone, une composition chimique, une concentration anesthésique, une pression partielle anesthésique, ou une combinaison comprenant au moins un parmi les éléments précédents provenant de la source de fluide ;
un oxygénateur (11) relié fluidiquement à la source de fluide, l'oxygénateur (11) étant configuré pour avoir une pression subatmosphérique, et l'oxygénateur (11) étant configuré pour oxygéner le sang et/ou d'autres fluides à partir d'une source de fluide séparée ;
un réservoir facultatif (6) de gaz de balayage relié fluidiquement à la source de fluide et à l'oxygénateur (11) ;
un capteur (10) de débit d'entrée de l'oxygénateur connecté fluidiquement à l'oxygénateur (11), le capteur (10) de débit d'entrée de l'oxygénateur étant configuré pour mesurer un ou plusieurs éléments parmi un débit, une composition de fluide, une pression, une température, une fraction d'oxygène, une fraction de dioxyde de carbone, une composition chimique, une concentration anesthésique, une pression partielle anesthésique ou une combinaison comprenant au moins l'un des éléments précédents provenant du réservoir de gaz de balayage lorsqu'il est présent ou d'un vaporisateur anesthésique ou de la source de fluide ;
un dispositif (9) de commande de débit d'entrée de l'oxygénateur connecté fluidiquement au réservoir de gaz de balayage (6) lorsqu'il est présent ou au vaporisateur anesthésique ou à la source de fluide, le dispositif (9) de commande de débit d'entrée d'oxygénateur étant configuré pour commander le débit de fluide provenant du réservoir de gaz de balayage lorsqu'il est présent ou du vaporisateur anesthésique ou de la source de fluide, le capteur (10) de débit d'entrée de l'oxygénateur étant situé entre l'oxygénateur (11) et le dispositif (9) de commande de débit d'entrée de l'oxygénateur ;
un capteur facultatif (19) de débit de sortie, connecté fluidiquement à l'oxygénateur, le capteur (19) de débit de sortie étant configuré pour mesurer un débit, une composition de fluide, une pression, une température, une fraction d'oxygène, une fraction de dioxyde de carbone, une composition chimique, une concentration anesthésique, une pression partielle anesthésique ou une combinaison comprenant au moins l'un des éléments précédents provenant du fluide oxygéné ;
un dispositif (18) de commande de débit de sortie relié fluidiquement à l'oxygénateur (11), le dispositif de commande de débit de sortie étant configuré pour commander le débit de fluide provenant de l'oxygénateur (11) ;
un dispositif de commande (22) configuré pour commander le dispositif (2) de commande de débit d'entrée, le dispositif (9) de commande de débit d'entrée de l'oxygénateur , ou le dispositif (18) de commande de débit de sortie, ou une combinaison comprenant au moins un parmi ce qui précède, en réponse à une mesure provenant du capteur d'entrée (3), du capteur (10) de débit d'entrée de l'oxygénateur, du capteur de sortie (19) lorsqu'il est présent, ou d'une combinaison comprenant au moins un des éléments précédents ; et
une source de vide (21) en aval de l'oxygénateur (11).

2. Le système selon la revendication 1, dans lequel l'oxygénateur (11) est un oxygénateur à membrane, un oxygénateur à membrane à diffusion, ou un oxygénateur à membrane microporeuse à fibres creuses.

3. Le système selon l'une quelconque des revendications 1 à 2, dans lequel l'oxygénateur (11) est un oxygénateur à membrane microporeuse avec un boîtier étanche.

4. Le système selon l'une quelconque des revendications 1 à 3, dans lequel l'oxygénateur (11) comprend un collecteur de sortie de gaz de balayage qui comprend des ouvertures d'évent.

5. Le système selon la revendication 4, dans lequel une ouverture est obstruée par une ceinture spécialement ajustée en matériau de qualité médicale tel que du silicone, du caoutchouc ou du plastique qui n'interfère pas avec les fixations de l'oxygénateur avec d'autres dispositifs.

6. Le système selon l'une quelconque des revendications 1 à 5, dans lequel l'oxygénateur (11) comprend en outre un boîtier, le boîtier de l'oxygénateur étant scellé en utilisant une chambre appliquée extérieurement ressemblant à une botte ou une boîte en matériau de qualité médicale tel que du silicone, du plastique, du polycarbonate, un polymère, du verre, du métal ou du caoutchouc.

7. Le système selon l'une quelconque des revendications 1 à 6, dans lequel le fluide est de l'oxygène, du dioxyde de carbone, un anesthésique, de l'air, de l'azote ou une combinaison comprenant au moins un parmi ceux-ci, et dans lequel le sang circule dans le système.

8. Le système selon l'une quelconque des revendications 1 à 7, dans lequel la source de fluide est un mélangeur configuré pour combiner un ou plusieurs fluides.

9. Le système selon l'une quelconque des revendications 1 à 8, dans lequel l'oxygénateur (11) comprend en outre un orifice de sortie de condensat ou une cavité de condensation pour collecter le condensat, l'orifice de sortie de condensat comprenant un trou de drainage, ou un réservoir séparé, la cavité de condensation comprenant un diaphragme perçable.

10. Le système selon l'une quelconque des revendications 1 à 9, dans lequel l'oxygénateur (11) comprend en outre une soupape de libération de surpression (15), qui est reliée fluidiquement à une sortie de l'oxygénateur.

11. Le système selon l'une quelconque des revendications 1 à 10, dans lequel au moins un parmi le dispositif (2) de commande de débit d'entrée, le dispositif (9) de commande de débit d'entrée de l'oxygénateur et le dispositif (18) de commande de débit de sortie est un dispositif de commande du débit massique, un dispositif de commande de débit à solénoïde ou une vanne à pointeau, configurés pour recevoir un signal provenant du dispositif de commande (22).

12. Le système selon l'une quelconque des revendications 1 à 11, comprenant en outre un régulateur de vide relié fluidiquement à l'oxygénateur, le régulateur de vide étant fixé à la source de vide (21) pour fournir la pression subatmosphérique.

13. Le système selon l'une quelconque des revendications 1 à 11, dans lequel le dispositif de commande (22) est configuré pour déterminer la différence entre une propriété mesurée au niveau de deux ou plusieurs des capteurs d'entrée (3), du capteur (10) de débit d'entrée de l'oxygénateur et le capteur (19) de débit de sortie, et est configuré pour ajuster le débit au niveau d'un ou plusieurs dispositifs (2) de commande de débit d'entrée, du dispositif (9) de commande de débit d'entrée de l'oxygénateur ou du dispositif (18) de commande de débit de sortie en réponse à la différence.
